# EUROPEAN PATENT SPECIFICATION

(11) **EP 4 501 366 B1**
(45) Date of publication and mention of the grant of the patent: **23.07.2025**
(21) Application number: 23189301.7
(22) Date of filing: 02.08.2023
(51) Int. Cl.: A61L 2/07

(54) **PROCESS AND DEVICE FOR STEAM STERILIZATION OF PRIMARY PACKAGING MEANS**
VERFAHREN UND VORRICHTUNG ZUR DAMPFSTERILISATION VON PRIMÄRPACKMITTELN
PROCÉDÉ ET DISPOSITIF DE STÉRILISATION À LA VAPEUR DE MOYENS D'EMBALLAGE PRIMAIRES

(43) Date of publication of application: 05.02.2025
(73) Proprietor: SCHOTT Pharma Schweiz AG, 9000 St. Gallen (CH); SCHOTT Pharma AG & Co. KGaA, 55122 Mainz (DE)
(72) Inventor: Breveglieri, Francesca, 8006 Zürich (CH); Henze, Inka, 55268 Nieder-Olm (DE); Rebrina, Aleksandra, 9300 Wittenbach (CH)
(74) Representative: Fuchs Patentanwälte Partnerschaft mbB

(56) References cited:
- WO-A2-2018/029590
- US-A- 3 598 516
- US-A- 5 277 875
- US-B2- 11 432 571

## Description

The present disclosure relates to a process and device for steam sterilization of primary packaging means as well as to a primary packaging means processed by the process. Primary packaging means, such as cartridges, vials, syringes, ampules, or other containers, may be used for pharmaceutical and cosmetic packaging applications. The sterilization process provides reduced water marks on the corresponding primary packaging means.

### Background

Steam sterilization is state of the art for many pharmaceutical and cosmetic products and their respective primary packaging means. These primary packaging means may be, for example, cartridges, vials, syringes, or ampules which can be made of a polymer, commonly COP or COC, or of glass. However, there exists a particular problem in the formation of water marks on the surface of these primary packaging means during the sterilization process. This applies to both types of materials but is in general more pronounced with glass.

An analysis of the water marks brought the result that they may comprise in particular silicone residues with different molecular weights and/or borates, sodium, calcium, sulfur salts, and nitrates, that characterize the glass components. In case of glass materials, they may mainly be attributed to evaporates from the hot forming process and are known as smelter dust.

Particularly for sterile pharmaceutical primary packaging means in the form of so called RTU (ready to use) containers, a typical packaging configuration for the transport from a manufacturer of pharmaceutical primary packaging means to a pharmaceutical company using the same comprises a tub or tray holding a multitude of the primary packaging means which is sealed with a non-woven. The non-woven is usually a flash spun plexifilamentary film-fibril structure made of high density polyethylene, which is known under the trademark Tyvek^{®} of the company DuPont, or a spunbonded very fine filaments non-woven. There are of course further applicable non-wovens of other companies. These selectively permeable non-wovens allow for the sterilization by means of gas or vapor, like ethylene oxide, steam, or hydrogen peroxide, even after closing the packaging. While being permeable for the sterilizing gas or vapor, the selectively permeable non-woven is essentially impermeable for microbes and thus forms a microbial barrier. The term "essentially impermeable for microbes" within this context refers to a Microbial Barrier measured according to ASTM F1608-16 of 2 to 6 Log Reduction Value (LRV).

The standard steam sterilization processes for solid products cannot be used without an increased risk of the loss of the integrity of the sterile barrier (tub/Tyvek^{®} seal seam) with the packaging configuration used in the market for RTU containers, particularly when made of glass. The standard process is characterized by a large number of vacuum pulses to remove trapped air, which might hinder effective steam penetration to the product and, consequently, efficient sterilization. A frequent and fast pressure variation results in high stress on the seal seam between the tub and the Tyvek^{®} layer because the pressure exchange through the non-woven Tyvek^{®} membrane is slower than in the chamber. The slower pressure equilibration inside the tub promotes a ballooning of the Tyvek^{®} seal (when pressure is decreased) or its adhesion to the packaged items (when pressure is increased). Additionally, the sterilization step is usually carried out at an increased pressure which is reduced back to ambient pressure after the sterilization process. Since the seal seam between the tub and the Tyvek^{®} layer is obtained by hot melting of a glue with a softening point close to steam sterilization conditions, the seal seam does not withstand the stress of pressure variation at high temperatures.

Another disadvantage for the RTU containers is that the saturated steam penetrates in the tub and condensates on surfaces, such as the nest, the glass or polymer, and the tub or bag, so that the tub may contain a high load of water which needs to be removed at the end of the sterilization procedure.

Document WO 2018/029590 A2 discloses a steam sterilization procedure for glass articles which uses a pulsed feed of steam for a reduction of white patches on the glass and damage to the physical and chemical structure of the glass. The steam flow inside the treatment chamber is oscillating in temperature and pressure and, thus, never has a constant mass. It is underlined that there are no sterilization steps in which pressure and/or temperature reach static sterilization steps and that the system is an open mass system. The pressure is varied in a range of from 0.2 bar to 1.15 bar. These process conditions have different drawbacks. Since there are constant pressure changes in the chamber, an eventual tub/Tyvek^{®} seal seam is under constant stress. This poses an increased risk of damage to the sterile barrier and creates the need to seal and sterilize the tubs anew or even to discard the glass articles in case of failure. Moreover, the pressure predominantly in the sub-ambient range will cause a lower steam temperature which in turn requires considerably increased sterilization times in order to achieve the mandatory sterilization level. With a suggested pulse duration of 24 min ± 1.5 min, the example graphs show a duration of about 450 min for a complete sterilization cycle.

Further, document US 3,598,516 A discloses a sterilizing process which is carried out in a sealed container or autoclave wherein air in the chamber is displaced with steam while the chamber is maintained at vacuum condition. The steam is fed in at a rate no greater than the capacity of the vacuum pump so that the pump will maintain the desired pressure in the chamber. A needle valve in the steam line is used to set the approximate flow rate. An additional solenoid valve is placed in the steam line in series with the needle valve and a thermostatic control is placed in the chamber drain line to turn the solenoid valve on and off to maintain the desired load temperature at approximately 130 °F (54 °C). The turning on and off of the solenoid valve to modulate the drain line temperature sets up a pulsing flow of steam and steam pressure condition of approximately 25 to 30 in. Hg (847 hPa to 1,016 hPa) which helps to purge the load of entrained air thereby heating the load more rapidly. Following the simultaneous application of vacuum and steam, a microbiocidal chemical sterilizing gas is admitted to the sterilizing chamber.

Hence, it is an object of the present invention to provide a steam sterilization process and a corresponding device which overcome at least one of the drawbacks of the prior art. In particular, the formation of water marks on the primary packaging means, in particular on the inner and/or outer surface of the primary packaging means, should be avoided or at least reduced while maintaining the sterility level of the sterilized containers.

### Summary of the disclosure

In a first aspect, the disclosure relates to a process for steam sterilization of primary packaging means in a steam sterilization device comprising the steps of
a) pre-heating the primary packaging means in the steam sterilization device to a temperature Tₚ which is at least equal to 15 °C below a dew point temperature T_{dp} of the steam at a predetermined pressure pₛ and predetermined temperature Tₛ at which the steam sterilization is to be performed, preferably at least equal to a dew point temperature T_{dp} of the steam at a predetermined pressure pₛ and predetermined temperature Tₛ at which the steam sterilization is to be performed, and/or which is at least 50 °C;
b) adjusting the pressure and temperature in the steam sterilization device to the predetermined pressure pₛ and predetermined temperature Tₛ and performing the sterilization by supplying steam to the steam sterilization device for at least a time tₛ sufficient to ensure a sterility assurance level of equal to or less than 10⁻⁶ according to Ph. Eur. 5.1.1; and
c) in a drying phase
   reducing a relative humidity in the steam sterilization device to < 50 % and
   either maintaining a drying pressure in the steam sterilization device constant to the predetermined pressure pₛ or setting the drying pressure in the steam sterilization device slightly lower than the predetermined pressure pₛ,
   before adjusting in a cooling phase the pressure and temperature in the steam sterilization device to ambient pressure and a temperature of ≤ 75 °C.

Setting a drying pressure in the steam sterilization device slightly lower than the predetermined pressure pₛ can mean that the pressure is reduced from pₛ by at most 75 hPa or at most 50 hPa or at most 25 hPa and/or by at least 5 hPa or at least 10 hPa or at least 20 hPa.

In a further aspect, the disclosure relates to a device for steam sterilization of primary packaging means according to claim 12.

A primary packaging means can be a cartridge, vial, syringe, ampule, or other container. It can for example be used for pharmaceutical and cosmetic packaging applications.

The primary packaging means can have a sterility assurance level of equal to or less than 10⁻⁶ according to Ph. Eur. 5.1.1.

The primary packaging means can be optically defect free, in other words not visible, in terms of water marks according to Ph. Eur. 2.9.20.

The primary packaging means can be at least partially coated on either the inner and/or outer surface.

Water marks in the sense of this disclosure can be residuals that appear on the surface of sterilized containers when sterilization and front-end processes are not properly optimized. They can usually appear in the form of more or less prominent irregular white or whitish spots with a typical size range of 0.005 - 0.01 mm² and eventually even up to about 7 mm². They can comprise silicone residues with different molecular weights and/or borates, sodium, calcium, sulfur salts, nitrates, and/or other glass components.

### Details of the disclosure

The details of this disclosure relate to the aspects described in the summary of disclosure. Any of the features of the embodiments described hereinafter may relate to one or more of the processes for steam sterilization as well as the device for steam sterilization as well as for the primary packaging means processed with steam sterilization.

When referring to "Ph. Eur. 5.1.1", this refers to chapter 5.1.1 of the 11^{th} edition of the European Pharmacopoeia defining sterilization methods for medical products and the sterility assurance level to be reached. Sterility is defined there as the absence of viable micro-organisms, as expressed by a sterility assurance level equal to or less than 10⁻⁶. Hence, Ph. Eur. 5.1.1 requires demonstrating the sterility assurance level of 10⁻⁶ through the correlation of pressure, time, and temperature. This is performed by measuring the temperature in the chamber and in the product, together with the biological assessment using suitable biological indicators during validation.

When referring to "Ph. Eur. 2.9.20", this refers to chapter 2.9.20 of the 11^{th} edition of the European Pharmacopoeia defining particulate contamination by visible particles.

The inventors have surprisingly found that simply reducing the contamination by silicone residues and smelter dust on the outside and/or inside of the packaging means in a washing step conducted before the sterilization procedure, which washing step can include washing inner and/or outer surfaces of the packaging means, will not be sufficient for an effective suppression of the water marks. Only a mitigation of the occurrence of the water marks upon water condensation and drying in the steam sterilization process was achieved.

After further investigation, another issue has been identified by the inventors which relates to the sterilization phase. At the end of the sterilization phase, the tub might be effectively dried by reducing the pressure that promotes water evaporation. However, this would risk the loss of the sterility barrier integrity due to the softened tub/Tyvek^{®} seal seam. Moreover, a decrease in temperature to further below the softening point of the glue is also not possible because the lower temperature would immediately foster condensation which must be avoided for several reasons. Condensing water collects contamination on the surface and concentrates it in a small area during the drying step, which leads to visual marks. Further, organic and silicone contamination constitute hydrophobic surfaces that lead to condensation of humidity in water droplets. Moreover, condensation leads to an increase of necessary drying time after sterilization. A solution to this problem of condensation has been found in separated drying and cooling phases.

First, in the drying phase the relative humidity in the chamber is reduced from about 100 % to < 50 % while the drying pressure in the steam sterilization device is maintained constant or set slightly lower than the predetermined pressure pₛ (sterilization pressure). Only subsequently in the cooling phase the temperature and the pressure are actively decreased. The relative humidity in the chamber may be reduced from about 100 % to < 50 % or from about 100 % to < 40 % or from about 100 % to < 30 %. By maintaining the pressure substantially constant or setting the drying pressure in the steam sterilization device slightly lower than the predetermined pressure pₛ (sterilization pressure), steam can be released, and thereby relative humidity can be reduced, while avoiding ingress of a large amount of compressed air. Thereby, temperature drop in the steam sterilization device can be minimized so as to avoid cooling down the primary packaging means too much and too fast. In particular, by setting the drying pressure slightly lower than the predetermined pressure pₛ a temperature drop of the primary packaging means below the chamber temperature can be avoided so as to avoid condensation thereon. Setting a drying pressure in the steam sterilization device slightly lower than the predetermined pressure pₛ can mean that the pressure is reduced from pₛ by at most 75 hPa or at most 50 hPa or at most 25 hPa and/or by at least 5 hPa or at least 10 hPa or at least 20 hPa.

To the further surprise of the inventors, it turned out that even a combination of the washing step described above with the separated drying and cooling phases will not be sufficient for an effective suppression of the water marks.

After further investigation they have identified another related issue which is the condensation of water on the colder surfaces of the primary packaging means and an eventual tub, tray, or nest when the warmer saturated steam gets in contact with it and when the product is cooled down after sterilization. These condensates that are present on the surfaces of the primary packaging already before sterilization caused an evaporative cooling in the tub and created a temperature drop of about 25 °C below 100°C which again created water marks. The effect of steam condensation becomes noticeable only when directly looking at the glass or polymer components. The impurities are collected in the water drops that leave visual defects (stains) on the glass upon drying.

By measuring the heating rates of RTU containers and tubs containing them in a heated oven that was pre-heated to 100 °C, it was found that these are very small (typically ≤ 1 °C/min). Such a heating rate is much smaller than that of the media in a sterilization chamber, i.e., in general air and steam. The heating rates slightly vary with the RTU containers and tubs. Tubs with higher weight, particularly with heavier glass primary packaging means, warm up slower than tubs with a lower weight, namely with lighter glass primary packaging means. The heating time of a common standard process typically is between 10 min and 20 min. Hence, the resulting temperature in a tub will be below 40 °C - 50 °C. When after the heating phase and the vacuum pulse the saturated steam enters the chamber, it will get in contact with the colder surfaces which consequently causes condensation in and on the tub. This condensed water cannot be taken up by the steam, as this is saturated, and the condensed water will remain inside the tub over the sterilization process.

To avoid water condensation in the pre-treatment, the RTU containers and tubs of this disclosure are sufficiently heated up in a first phase. The temperature Tₚ to which they are heated is at least equal to the 15 °C below dew point temperature T_{dp} of the steam at the conditions of the steam sterilization, preferably at least equal to a dew point temperature T_{dp} of the steam at a predetermined pressure pₛ and predetermined temperature Tₛ at which the steam sterilization is to be performed, and/or is at least 50 °C. If they have at least the dew point temperature, the steam will no longer condense on their surfaces. As a general rule it has been found that a temperature of at least 50 °C may be used. The temperature may be at least 50 °C, at least 60 °C, at least 70 °C, at least 80 °C, or at least 85 °C. The temperature may be at most the temperature at which the steam sterilization is to be performed. The temperature may be chosen depending on the type of the primary packaging means. For example, for certain primary packaging means a minor pre-heating to a temperature of at least 50 °C or at least 60 °C may be sufficient, whereas for other primary packaging means a higher pre-heating to a temperature of at least 70°C, at least 80 °C, or even at least 85 °C may be required. With this approach, the condensation before sterilization can be minimized or avoided.

Since these higher temperatures in the pre-treatment phase again result in a softening of the tub/Tyvek^{®} seal seam, a deep vacuum pulse should be avoided in order not to weaken the sterile barrier. Consequently, more air may be present in the tub which might potentially hinder the product sterilization. Depending on the applied pressure level, a corresponding moderate increase in the sterilization time may ensure the desired sterility level.

A combination of the separated drying and cooling phases with this avoiding or minimizing of water condensation before the sterilization step finally proved itself to be suitable for achieving an effective suppression of the water marks while keeping the sterility level intact. A washing step as described above may be installed in addition to these two measures but is insufficient when used in combination with either of them alone.

In embodiments, the primary packaging means may be further packaged in a secondary packaging container, such as a tub or tray, sealed with a non-woven, wherein the secondary packaging container optionally includes a nest and/or optionally holds a multitude of the primary packaging means. The disclosed process may be used with the primary packaging means directly placed in a suitable device for steam sterilization or with the common packaging for RTU containers in the form of a tub sealed with a non-woven, in particular with Tyvek^{®}.

In embodiments, the pre-heating in step a) may executed for 0.5 h to 3 h or for 0.75 h to 2.5 h or for 1 h to 2 h. The pre-heating in step a) may executed for at least 0.5 h or for at least 0.75 h or for at least 1 h. The pre-heating in step a) may executed for at most 3 h or for at most 2.5 h or for at most 2 h. These times for pre-heating have been found to generally provide enough time for the common primary packaging means to sufficiently get heated to the core.

In embodiments, in step a) the primary packaging means can be pre-heated to a temperature Tₚ of at least 50 °C or at least 60 °C or at least 70 °C or at least 85 °C. In step a) the primary packaging means can be pre-heated to a temperature Tₚ of at most the temperature Tₛ at which the steam sterilization is to be performed or of at most Tₛ - 5 °C or of at most Tₛ - 10 °C or of at most Tₛ - 15 °C or of at most Tₛ - 20 °C or of at most Tₛ - 25 °C or of at most 100 °C. These temperatures have been found to be suitable for preventing or reducing condensation on the surfaces of the primary packaging means sufficient for preventing the formation of water marks in combination with the other measures taken.

In embodiments, the steam sterilization in step b) may be executed for a time tₛ of at least 1.5 h or at least 1.0 h or at least 0.75 h or at least 0.5 h or at least 0.3 h and/or at a temperature Tₛ in the range of from 120 °C to 127 °C or from 120 °C to 125 °C or from 121 °C to 124 °C. The steam sterilization in step b) may be executed for a time tₛ of at most 5 h or of at most 4 h of at most 3 h. This has been found to reliably provide the common sterilization level of 10⁻⁶.

In embodiments, the steam sterilization in step b) may be executed at a pressure pₛ which is above ambient pressure, in particular in a range from 1,100 hPa to 2,500 hPa or in a range from 1,400 hPa to 2,400 hPa or in a range from 1,800 hPa to 2,300 hPa. The steam sterilization in step b) may be executed at a pressure pₛ which at least 1,100 hPa or at least 1,400 hPa or at least 1,800 hPa. The steam sterilization in step b) may be executed at a pressure pₛ which at most 2,500 hPa or at most 2,400 hPa or at most 2,300 hPa. A sterilization in moderate support pressure conditions provides an optimum of efficiency and required treatment time and facilitates final drying at the end of the sterilization process by reducing the pressure. Moderate support pressure can be approx. 100 hPa above an absolute saturated steam pressure. For example, moderate support pressure can be approx. 2,200 hPa for saturated steam pressure of about 2,100 hPa absolute.

In embodiments, the adjusting of the pressure in the cooling phase in step c) may be executed at a rate of at most 500 hPa/min or at most 250 hPa/min or at most 100 hPa/min or at most 75 hPa/min or at most 50 hPa/min. If the rate is selected too high the stress on the sealing seam of the non-woven might cause a loss of integrity.

In embodiments, between step a) and step b) the pressure in the steam sterilization device may be reduced to a range of 100 hPa to 1,000 hPa or reduced to a range of 250 hPa to 1,000 hPa or reduced to a range of 500 hPa to 1,000 hPa or reduced to a range of 700 hPa to 1,000 hPa or reduced to a range of 800 hPa to 975 hPa or reduced to a range of 900 hPa to 975 hPa, optionally at a rate of at most 500 hPa/min or at most 250 hPa/min or at most 100 hPa/min or 75 hPa/min or at most 50 hPa/min or at most 25 hPa/min. The pressure in the steam sterilization device may be reduced to not less than 100 hPa or reduced to not less than 250 hPa or reduced to not less than 500 hPa or reduced to not less than 700 hPa or reduced to not less than 800 hPa or reduced to not less than 900 hPa. The pressure in the steam sterilization device may be reduced to not more than 975 hPa or not more than 950 hPa or not more than 925 hPa or not more than 900 hPa. This helps reducing the stress on the sealing seam of the non-woven, in particular when adjusted at the mentioned rates.

In embodiments, during the drying phase in step c) the temperature in the steam sterilization device may be reduced from Tₛ to a temperature in the range of from 120 °C to 90 °C or from 115 °C to 95 °C or from 110 °C to 100 °C and/or the pressure in the steam sterilization device is held constant or only slightly lower than the predetermined pressure pₛ (sterilization pressure). The temperature in the steam sterilization device may be reduced from Tₛ to a temperature of at most 120 °C or at most 115 °C or at most 110 °C. The temperature in the steam sterilization device may be reduced from Tₛ to a temperature of at least 90 °C or at least 95 °C or at least 100 °C. At the beginning of the drying phase, temperature can be at least 100 °C. Temperature can be reduced by setting the drying pressure in the steam sterilization device slightly lower than the predetermined pressure pₛ (sterilization pressure), steam can be released, and thereby relative humidity can be reduced, while avoiding ingress of a large amount of compressed air. Thereby, temperature drop in the steam sterilization device can be minimized so as to avoid cooling down the primary packaging means too much and too fast. In particular, by setting the drying pressure slightly lower than the predetermined pressure pₛ a temperature drop of the primary packaging means below the chamber temperature can be avoided so as to avoid condensation thereon. Setting a drying pressure in the steam sterilization device slightly lower than the predetermined pressure pₛ can mean that the pressure is reduced from pₛ by at most 75 hPa or at most 50 hPa or at most 25 hPa and/or by at least 5 hPa or at least 10 hPa or at least 20 hPa.

In embodiments, between step b) and step c) the pressure in the steam sterilization device may be reduced from pₛ by at most 75 hPa or at most 50 hPa or at most 25 hPa. A slight reduction of the pressure before initiating the drying phase may be beneficial for drying. An only slight reduction of the pressure before initiating the drying phase may be beneficial for avoiding large temperature drop due to introduction of too much compressed air.

In embodiments, before step a) an additional washing step may be executed comprising a rinsing of the outer surface of the primary packaging means with water and/or a polar organic solvent. As explained above, such a washing step alone will not be sufficient to avoid the formation of water marks. However, it can be used as an additional step to reduce the surface contamination and in doing so improve the reduction or elimination of water marks. Rinsing with water or polar organic solvents, such as alcohols, esters, and ethers, is most effective due to the source of the contamination being salts from the smelter dust and silicones.

In embodiments, the device may for this purpose further comprise a washing station for rinsing of the outer surface of the primary packaging means with water and/or a polar organic solvent.

### Brief description of the Figures

- **Figure 1**: shows a schematic view of a device for steam sterilization.
- **Figure 2**: shows a schematic diagram of the temperature and pressure during a steam sterilization process.

### Description of the Figures

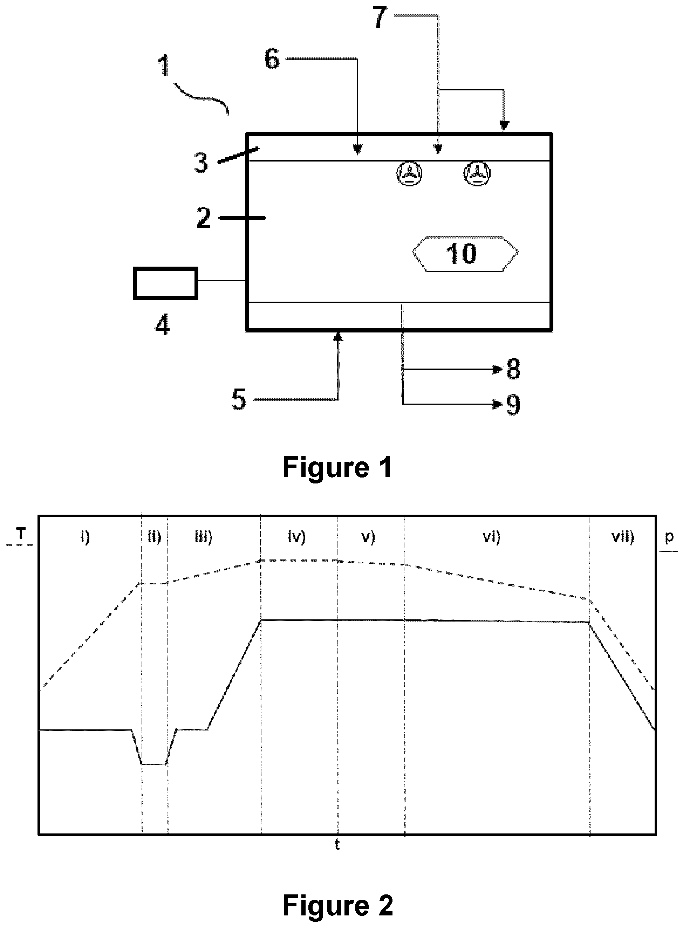

**Figure 1** shows a schematic view of a device 1 for steam sterilization. The device 1 comprises a chamber 2 for receiving the primary packaging means to be sterilized. In the figure, they are shown in a secondary packaging container 10 which is sealed with a non-woven layer. The chamber 2 is surrounded by a heating and cooling jacket 3 which enables a programmed heating and cooling of the chamber 2 by means of the controller 4 for temperature and pressure. The heating and cooling jacket 3 is connected to a supply of cooling water 5 for cooling and a supply of steam 7 for heating. The chamber 2 is connected to a supply of steam 7, a supply of pressurized air 6, and a vacuum pump 8. The supply of pressurized air 6 may provide hot or cold air and can further be used to set the temperature and pressure in the chamber 2. Condensed water may be removed from the chamber 2 through the drain 9. Further indicated are two ventilators of the chamber 2 which help to homogenize the atmosphere within the chamber 2.

The controller 4 for temperature and pressure within the chamber 2 and of the supply of steam 7 and the supply of pressurized air 6 is configured to perform a steam sterilization process according to this disclosure.

**Figure 2** shows a schematic diagram of the temperature and pressure during a steam sterilization process according to this disclosure. The temperature is shown by the dashed line and pressure by the solid line. Indicated by the vertical dashed lines are seven program steps i) to vii) of the steam sterilization process. The presented example uses a pre-vacuum of 900 hPa and an increased pressure of 2,200 hPa for the sterilization phase of cartridges made of glass as the primary packaging means which are placed in a tub sealed with a non-woven as the secondary packaging container 10. All pressure changes were made at a rate of 50 hPa/min.

In pre-heating step i), the chamber 2 with the primary packaging means within the secondary packaging container 10 is pre-heated at constant pressure to a temperature Tₚ of, for example, 85 °C for 1.5 h. At the end of step i), the pressure is reduced to 900 hPa.

In pre-vacuum step ii), the pressure and temperature are held constant for a time sufficient to equilibrate the pressure inside the secondary packaging container 10, such as 5 min.

In the pressurization step iii), heating of the chamber 2 is started, and the pressure is first increased back to ambient pressure, held constant for a time, such as 5 min, and finally increased to the sterilization pressure pₛ, i.e., in this example 2,200 hPa. The set temperature Tₛ may, for example, be 122 °C.

In sterilization step iv), the system may again be given some time, such as 5 min, to equilibrate before the sterilization treatment is performed at constant pressure and temperature for, for example, 1 h in order to reach the sterilization level of 10⁻⁶.

In the drying step v), while the pressure is constant, the relative humidity is decreased to slightly above 30 % while the heating is still turned on. Hence, only a slight decrease in temperature occurs in this step which may be executed, for example, for 30 min.

In the drying and cooling step vi), the relative humidity is further slightly decreased, stabilizing in this example around a constant value of about 30 %. The heating is deactivated in this step which may be executed, for example, for 60 min. The temperature may be decreased to 110 °C, whereas the pressure is still held constant.

In the final cooling and depressurization step vii), the cooling is activated, and the pressure is released. At the end of the step, the pressure is back to ambient pressure and the temperature below 75 °C. In the shown example, the temperature is also brought back to ambient temperature of about 23 °C.

The cartridges were examined at the end of the sterilization process and found to be free of water marks.

### Reference numerals

- 1: device for steam sterilization
- 2: chamber
- 3: heating and cooling jacket
- 4: controller for temperature and pressure
- 5: supply of cooling water
- 6: supply of pressurized air
- 7: supply of steam
- 8: vacuum pump
- 9: drain
- 10: secondary packaging container

## Claims

1. Process for steam sterilization of primary packaging means in a steam sterilization device (1) comprising the steps of
a) pre-heating the primary packaging means in the steam sterilization device (1) to a temperature Tₚ which is at least equal to 15 °C below a dew point temperature T_{dp} of the steam at a predetermined pressure pₛ and predetermined temperature Tₛ at which the steam sterilization is to be performed, preferably at least equal to a dew point temperature T_{dp} of the steam at a predetermined pressure pₛ and predetermined temperature Tₛ at which the steam sterilization is to be performed, and/or which is at least 50 °C;
b) adjusting the pressure and temperature in the steam sterilization device (1) to the predetermined pressure pₛ and predetermined temperature Tₛ and performing the sterilization by supplying steam to the steam sterilization device (1) for at least a time tₛ sufficient to ensure a sterility assurance level of equal to or less than 10⁻⁶ according to Ph. Eur. 5.1.1; and
c) in a drying phase
reducing a relative humidity in the steam sterilization device (1) to < 50 % and
either maintaining a drying pressure in the steam sterilization device constant to the predetermined pressure pₛ or reducing the drying pressure in the steam sterilization device from the predetermined pressure pₛ by at most 75 hPa or at most 50 hPa or at most 25 hPa and/or by at least 5 hPa or at least 10 hPa or at least 20 hPa,
before adjusting in a cooling phase the pressure and temperature in the steam sterilization device (1) to ambient pressure and a temperature of ≤ 75 °C.

2. Process for steam sterilization according to claim 1, wherein the primary packaging means are further packaged in a secondary packaging container (10), such as a tub or tray, sealed with a non-woven, wherein the secondary packaging container (10) optionally includes a nest and/or optionally holds a multitude of the primary packaging means.

3. Process for steam sterilization according to claim 1 or 2, wherein the pre-heating in step a) is executed for 0.5 h to 3 h or for 0.75 h to 2.5 h or for 1 h to 2 h.

4. Process for steam sterilization according to one of the preceding claims, wherein in step a) the primary packaging means is pre-heated to a temperature Tₚ of at least 60 °C or at least 70 °C or at least 85 °C.

5. Process for steam sterilization according to one of the preceding claims, wherein the steam sterilization in step b) is executed for a time tₛ of at least 1.5 h or at least 1.0 h or at least 0.75 h or at least 0.5 h or at least 0.3 h and/or at a temperature Tₛ in the range of from 120 °C to 127 °C or from 120 °C to 125 °C or from 121 °C to 124 °C.

6. Process for steam sterilization according to one of the preceding claims, wherein the steam sterilization in step b) is executed at a pressure pₛ which is above ambient pressure, in particular in a range from 1,100 hPa to 2,500 hPa or in a range from 1,400 hPa to 2,400 hPa or in a range from 1,800 hPa to 2,300 hPa.

7. Process for steam sterilization according to one of the preceding claims, wherein the adjusting of the pressure in the cooling phase in step c) is executed at a rate of at most 500 hPa/min or at most 250 hPa/min or at most 100 hPa/min or at most 75 hPa/min or at most 50 hPa/min.

8. Process for steam sterilization according to one of the preceding claims, wherein between step a) and step b) the pressure in the steam sterilization device (1) is reduced to a range of 100 hPa to 1,000 hPa or reduced to a range of 250 hPa to 1,000 hPa or reduced to a range of 500 hPa to 1,000 hPa or reduced to a range of 700 hPa to 1,000 hPa or reduced to a range of 800 hPa to 975 hPa or reduced to a range of 900 hPa to 975 hPa, optionally at a rate of at most 500 hPa/min or at most 250 hPa/min or at most 100 hPa/min or 75 hPa/min or at most 50 hPa/min or at most 25 hPa/min.

9. Process for steam sterilization according to one of the preceding claims, wherein during the drying phase in step c) the temperature in the steam sterilization device (1) is reduced from Tₛ to a temperature in the range of from 120 °C to 90 °C or from 115 °C to 95 °C or from 110 °C to 100 °C and/or the pressure in the steam sterilization device (1) is held constant.

10. Process for steam sterilization according to one of the preceding claims, wherein between step b) and step c) the pressure in the steam sterilization device (1) is reduced from pₛ by at most 75 hPa or at most 50 hPa or at most 25 hPa.

11. Process for steam sterilization according to one of the preceding claims, wherein before step a) an additional washing step is executed comprising a rinsing of the inner and/or outer surface of the primary packaging means with water and/or a polar organic solvent.

12. Device (1) for steam sterilization of primary packaging means, the device (1) comprising
- a chamber (2) for receiving the primary packaging means which is connected to a supply of steam (7), a supply of pressurized air (6), and a vacuum pump (8), and which is provided with a heating and cooling jacket (3); and
- a controller (4) for adjusting the temperature and pressure within the chamber (2) by means of the supply of steam (7) and the supply of pressurized air (6), which is configured to perform a steam sterilization process according to one of the preceding claims.

13. Device (1) for steam sterilization according to claim 12, further comprising a washing station for rinsing of the inner and/or outer surface of the primary packaging means with water and/or a polar organic solvent.

## Patentansprüche

1. Verfahren zur Dampfsterilisation von primären Verpackungseinrichtungen in einer Dampfsterilisationsvorrichtung (1), mit den Schritten:
a) Vorerwärmen der primären Verpackungseinrichtungen in der Dampfsterilisationsvorrichtung (1) auf eine Temperatur Tₚ, die mindestens 15°C unter einer Taupunkttemperatur T_{dp} des Dampfes bei einem vorgegebenen Druck pₛ und einer vorgegebenen Temperatur Tₛ liegt, bei denen die Dampfsterilisation ausgeführt werden soll, vorzugsweise mindestens gleich der Taupunkttemperatur T_{dp} des Dampfes bei dem vorgegebenen Druck pₛ und der vorgegebenen Temperatur Tₛ ist, bei der die Dampfsterilisation ausgeführt werden soll, und/oder die mindestens 50°C beträgt;
b) Einstellen des Drucks und der Temperatur in der Dampfsterilisationsvorrichtung (1) auf den vorgegebenen Druck pₛ und die vorgegebene Temperatur Tₛ und Ausführen der Sterilisation durch Zuführen von Dampf zur Dampfsterilisationsvorrichtung (1) für mindestens eine Zeit tₛ, die ausreicht, um ein Sterilitätssicherheitsniveau von kleiner oder gleich 10⁻⁶ gemäß Ph. Eur. 5.1.1 sicherzustellen; und
c) in einer Trocknungsphase:
Reduzieren einer relativen Feuchtigkeit in der Dampfsterilisationsvorrichtung (1) auf < 50%; und
entweder Aufrechterhalten eines Trocknungsdrucks in der Dampfsterilisationsvorrichtung konstant auf dem vorgegebenen Druck pₛ oder Reduzieren des Trocknungsdrucks in der Dampfsterilisationsvorrichtung von dem vorgegebenen Druck pₛ um höchstens 75 hPa oder höchstens 50 hPa oder höchstens 25 hPa und/oder um mindestens 5 hPa oder mindestens 10 hPa oder mindestens 20 hPa,
Einstellen des Drucks und der Temperatur in der Dampfsterilisationsvorrichtung (1) auf Umgebungsdruck und eine Temperatur von 75°C in einer Kühlphase.

2. Verfahren nach Anspruch 1, wobei die primären Verpackungseinrichtungen ferner in einem sekundären Verpackungsbehälter (10), wie beispielsweise einer Wanne oder Schale, die mit einem Vlies versiegelt ist, weiter verpackt werden, wobei der sekundäre Verpackungsbehälter (10) optional eine Nestung aufweist und/oder optional eine Vielzahl der primären Verpackungseinrichtungen hält.

3. Verfahren nach Anspruch 1 oder 2, wobei das Vorerwärmen in Schritt a) für 0,5 h bis 3 h oder für 0,75 h bis 2,5 h oder für 1 h bis 2 h ausgeführt wird.

4. Verfahren nach einem der vorangehenden Ansprüche, wobei in Schritt a) die primären Verpackungseinrichtungen auf eine Temperatur Tₚ von mindestens 60°C oder mindestens 70°C oder mindestens 85°C vorerwärmt werden.

5. Verfahren nach einem der vorangehenden Ansprüche, wobei die Dampfsterilisation in Schritt b) für eine Zeit tₛ von mindestens 1,5 h oder mindestens 1,0 h oder mindestens 0,75 h oder mindestens 0,5 h oder mindestens 0,3 h und/oder bei einer Temperatur Tₛ im Bereich von 120°C bis 127°C oder von 120°C bis 125°C oder von 121°C bis 124°C ausgeführt wird.

6. Verfahren nach einem der vorangehenden Ansprüche, wobei die Dampfsterilisation in Schritt b) bei einem Druck pₛ ausgeführt wird, der über dem Umgebungsdruck liegt, insbesondere in einem Bereich von 1100 hPa bis 2500 hPa oder in einem Bereich von 1400 hPa bis 2400 hPa oder in einem Bereich von 1800 hPa bis 2300 hPa.

7. Verfahren nach einem der vorangehenden Ansprüche, wobei das Einstellen des Drucks in der Kühlphase in Schritt c) mit einer Geschwindigkeit von höchstens 500 hPa/min oder höchstens 250 hPa/min oder höchstens 100 hPa/min oder höchstens 75 hPa/min oder höchstens 50 hPa/min ausgeführt wird.

8. Verfahren nach einem der vorangehenden Ansprüche, wobei zwischen Schritt a) und Schritt b) der Druck in der Dampfsterilisationsvorrichtung (1) auf einen Bereich von 100 hPa bis 1000 hPa oder auf einen Bereich von 250 hPa bis 1000 hPa oder auf einen Bereich von 500 hPa bis 1000 hPa oder auf einen Bereich von 700 hPa bis 1000 hPa oder auf einen Bereich von 800 hPa bis 975 hPa oder auf einen Bereich von 900 hPa bis 975 hPa reduziert wird, optional mit einer Geschwindigkeit von höchstens 500 hPa/min oder höchstens 250 hPa/min oder höchstens 100 hPa/min oder 75 hPa/min oder höchstens 50 hPa/min oder höchstens 25 hPa/min.

9. Verfahren nach einem der vorangehenden Ansprüche, wobei während der Trocknungsphase in Schritt c) die Temperatur in der Dampfsterilisationsvorrichtung (1) von Tₛ auf eine Temperatur im Bereich von 120°C bis 90°C oder von 115°C bis 95°C oder von 110°C bis 100°C reduziert wird und/oder der Druck in der Dampfsterilisationsvorrichtung (1) konstant gehalten wird.

10. Verfahren nach einem der vorangehenden Ansprüche, wobei zwischen Schritt b) und Schritt c) der Druck in der Dampfsterilisationsvorrichtung (1) von pₛ um höchstens 75 hPa oder höchstens 50 hPa oder höchstens 25 hPa reduziert wird.

11. Verfahren nach einem der vorangehenden Ansprüche, wobei vor Schritt a) ein zusätzlicher Waschschritt ausgeführt wird, der ein Spülen der Innen- und/oder Außenfläche der primären Verpackungseinrichtungen mit Wasser und/oder einem polaren organischen Lösungsmittel aufweist.

12. Vorrichtung (1) zur Dampfsterilisation von primären Verpackungseinrichtungen, wobei die Vorrichtung (1) aufweist:
eine Kammer (2) zum Aufnehmen der primären Verpackungseinrichtungen, die mit einer Dampfzufuhr (7), einer Druckluftzufuhr (6) und einer Vakuumpumpe (8) verbunden ist und einen Heiz- und Kühlmantel (3) aufweist; und
eine Steuereinheit (4) zum Einstellen der Temperatur und des Drucks in der Kammer (2) mittels der Dampfzufuhr (7) und der Druckluftzufuhr (6), die dafür konfiguriert ist, ein Dampfsterilisationsverfahren nach einem der vorangehenden Ansprüche auszuführen.

13. Vorrichtung (1) nach Anspruch 12, ferner mit einer Waschstation zum Spülen der Innen- und/oder Außenfläche der primären Verpackungseinrichtungen mit Wasser und/oder einem polaren organischen Lösungsmittel.

## Revendications

1. Procédé de stérilisation à la vapeur de moyens d'emballage primaire dans un dispositif de stérilisation à la vapeur (1) comprenant les étapes
a) préchauffer les moyens d'emballage primaire dans le dispositif de stérilisation à la vapeur (1) à une température Tₚ, qui est au moins égale à 15 °C en dessous d'une température de point de rosée T_{dp} de la vapeur à une pression prédéterminée Pₛ et à une température prédéterminée Tₛ à laquelle la stérilisation à la vapeur doit être effectuée, de préférence au moins égale à une température de point de rosée T_{dp} de la vapeur à une pression prédéterminée Pₛ et à une température prédéterminée Tₛ à laquelle la stérilisation à la vapeur doit être effectuée, et/ou qui est au moins égale à 50 °C ;
b) régler la pression et la température dans le dispositif de stérilisation à la vapeur (1) à la pression prédéterminée Pₛ et à la température prédéterminée Tₛ et effectuer la stérilisation en fournissant de la vapeur au dispositif de stérilisation à la vapeur (1) pendant au moins une durée tₛ suffisante pour garantir un niveau d'assurance de stérilité égal ou inférieur à 10⁻⁶ selon la Ph.Eur. 5.1.1 ; et
c) dans une phase de séchage
réduire une humidité relative dans le dispositif de stérilisation à la vapeur (1) à < 50 % et
soit maintenir une pression de séchage dans le dispositif de stérilisation à la vapeur constante à la pression prédéterminée Pₛ, soit réduire la pression de séchage dans le dispositif de stérilisation à la vapeur par rapport à la pression prédéterminée Ps d'au plus 75 hPa ou d'au plus 50 hPa ou d'au plus 25 hPa et/ou d'au moins 5 hPa ou d'au moins 10 hPa ou d'au moins 20 hPa, avant d'ajuster, dans une phase de refroidissement, la pression et la température dans le dispositif de stérilisation à la vapeur (1) à la pression ambiante et à une température ≤ 75 °C.

2. Procédé de stérilisation à la vapeur selon la revendication 1, dans lequel les moyens d'emballage primaire sont en outre emballé dans un contenant d'emballage secondaire (10), tel qu'une cuve ou un plateau, scellé avec un non-tissé, dans lequel le contenant d'emballage secondaire (10) comprend éventuellement un nid et/ou contient éventuellement une multitude des moyens d'emballage primaire.

3. Procédé de stérilisation à la vapeur selon la revendication 1 ou 2, dans lequel le préchauffage à l'étape a) est exécuté pendant 0,5 h à 3 h ou pendant 0,75 h à 2,5 h ou pendant 1 h à 2 h.

4. Procédé de stérilisation à la vapeur selon l'une des revendications précédentes, dans lequel, à l'étape a), le moyen d'emballage primaire est préchauffé à une température Tₚ d'au moins 60 °C ou d'au moins 70 °C ou d'au moins 85 °C.

5. Procédé de stérilisation à la vapeur selon l'une des revendications précédentes, dans lequel la stérilisation à la vapeur à l'étape b) est exécutée pendant une durée tₛ d'au moins 1,5 h ou d'au moins 1,0 h ou d'au moins 0,75 h ou d'au moins 0,5 h ou d'au moins 0,3 h et/ou à une température Tₛ comprise entre 120 °C et 127 °C ou entre 120 °C et 125 °C ou entre 121 °C et 124 °C.

6. Procédé de stérilisation à la vapeur selon l'une des revendications précédentes, dans lequel la stérilisation à la vapeur à l'étape b) est exécutée à une pression Pₛ supérieure à la pression ambiante, en particulier dans une plage allant de 1100 hPa à 2500 hPa ou de 1400 hPa à 2400 hPa ou de 1800 hPa à 2300 hPa.

7. Procédé de stérilisation à la vapeur selon l'une des revendications précédentes, dans lequel le réglage de la pression dans la phase de refroidissement à l'étape c) est effectué à une vitesse d'au plus 500 hPa/min ou d'au plus 250 hPa/min ou d'au plus 100 hPa/min ou d'au plus 75 hPa/min ou d'au plus 50 hPa/min.

8. Procédé de stérilisation à la vapeur selon l'une des revendications précédentes, dans lequel, entre l'étape a) et l'étape b), la pression dans le dispositif de stérilisation à la vapeur (1) est réduite à une plage de 100 hPa à 1000 hPa ou à une plage de 250 hPa à 1000 hPa ou à une plage de 500 hPa à 1000 hPa ou réduite à une plage de 700 hPa à 1000 hPa ou réduite à une plage de 800 hPa à 975 hPa ou réduite à une plage de 900 hPa à 975 hPa, éventuellement à une vitesse d'au plus 500 hPa/min ou d'au plus 250 hPa/min ou d'au plus 100 hPa/min ou 75 hPa/min ou d'au plus 50 hPa/min ou d'au plus 25 hPa/min.

9. Procédé de stérilisation à la vapeur selon l'une des revendications précédentes, dans lequel, pendant la phase de séchage à l'étape c), la température dans le dispositif de stérilisation à la vapeur (1) est ramenée de Ts à une température comprise entre 120 °C et 90 °C ou entre 115 °C et 95 °C ou entre 110 °C et 100 °C et/ou la pression dans le dispositif de stérilisation à la vapeur (1) est maintenue constante.

10. Procédé de stérilisation à la vapeur selon l'une des revendications précédentes, dans lequel, entre l'étape b) et l'étape c), la pression dans le dispositif de stérilisation à la vapeur (1) est réduite depuis Pₛ d'au plus 75 hPa ou d'au plus 50 hPa ou d'au plus 25 hPa.

11. Procédé de stérilisation à la vapeur selon l'une des revendications précédentes, dans lequel, avant l'étape a), une étape de lavage supplémentaire est exécutée, comprenant un rinçage de la surface intérieure et/ou extérieure des moyens d'emballage primaire avec de l'eau et/ou un solvant organique polaire.

12. Dispositif (1) pour la stérilisation à la vapeur d'un moyen d'emballage primaire, le dispositif (1) comprenant
- une chambre (2) destinée à recevoir les moyens d'emballage primaire, reliée à une alimentation en vapeur (7), à une alimentation en air sous pression (6) et à une pompe à vide (8), et pourvue d'une enveloppe de chauffage et de refroidissement (3) ; et
- un contrôleur (4) pour ajuster la température et la pression à l'intérieur de la chambre (2) au moyen de l'alimentation en vapeur (7) et de l'alimentation en air sous pression (6), qui est configuré pour effectuer un processus de stérilisation à la vapeur selon l'une des revendications précédentes.

13. Dispositif (1) de stérilisation à la vapeur selon la revendication 12, comprenant en outre une station de lavage pour le rinçage de la surface intérieure et/ou extérieure des moyens d'emballage primaire avec de l'eau et/ou un solvant organique polaire.
